# EUROPEAN PATENT APPLICATION

(11) **EP 3 406 259 A1**
(43) Date of publication of application: **28.11.2018**
(21) Application number: 17172659.9
(22) Date of filing: 24.05.2017
(51) Int. Cl.: A61K 38/18, A61P 27/16

(54) **NEUROTROPHINS FOR USE IN THE TREATMENT OF HEARING LOSS**

(71) Applicant: Dompé farmaceutici S.p.A., 20122 Milan (IT)
(72) Inventor: DE PIZZOL, Maria, I-20122 Milano (MI) (IT); ARAMINI, Andrea, I-67100 L'Aquila (AQ) (IT); SIRICO, Anna, I-80131 Napoli (NA) (IT); ZIPPOLI, Mara, I-80131 Napoli (NA) (IT)
(74) Representative: Pistolesi, Roberto

(57) **Abstract**

The present invention relates to the use of neurotrophins in the treatment of hearing disorders related to hearing loss using the transtympanic route of administration, in particular in the treatment of sudden deafness, blast-induced hearing loss, ototoxicity, ARHL (age related hearing loss) and noise damage. The invention further relates to the use of compositions containing neurotrophins, in particular NGF, in the treatment of hearing disorders.

## Description

The present invention relates to the use of neurotrophins in the treatment of hearing disorders related to hearing loss using the transtympanic route of administration, in particular in the treatment of sudden deafness, blast-induced hearing loss, ototoxicity, ARHL (age related hearing loss) and noise damage. The invention further relates to the use of compositions containing neurotrophins, in particular NGF, in the treatment of hearing disorders.

### BACKGROUND OF THE INVENTION

Deafness occurs in 0.1-0.2% of newborns, and hearing loss affects up to 70% of the population over the age of 75 years (Sprinzl GM et al.,Gerontology 2010;56(3):351-8). Sound signals are converted into electrical impulses by auditory hair cells located in the cochlea. Degeneration of the hair cells, due to infections, mechanical injury, aminoglycoside antibiotics, acoustic trauma, aging and chemical-induced ototoxicity, is a major cause of hearing impairments or loss (Delterne P et al., Acta Otolaryngol 1993; 113:312-377).

Ototoxic drugs include the commonly used aminoglycoside antibiotics, such as gentamicin, for the treatment of infections caused by Gram-negative bacteria, the widely used chemotherapeutic agent cisplatin and its analogues, quinine and its analogues, salycilate and its analogues, and loop-diuretics. The toxic effects of these drugs on auditory cells and spiral ganglion neurons are often the limiting factor for their therapeutic usefulness. For example, antibacterial aminoglycosides such as gentamicin and the like are known to have serious toxicity, particularly ototoxicity and nephrotoxicity, which reduce the usefulness of such antimicrobial agents (Goodman and Gilman's The Pharmacological Basis of Therapeutics, Vol 70, Issue 5, May 1981, page 581-6th ed.).

Otitis media is a term used to describe infections of the middle ear. Typically antibiotics are systemically administered for infections of the middle ear. Systemic administration of antibiotics generally results in a prolonged lag time to achieve therapeutic levels in the middle ear and requires high initial doses in order to achieve such levels. Systemic administration is most often effective when infection has reached advanced stages, but at this point permanent damage may already have been caused to the middle and inner ear structure. Clearly, ototoxicity is a dose-limiting side-effect of antibiotic administration.

Auditory apparatus can be divided into the external and middle ear, inner ear and auditory nerve and central auditory pathways. While having some variations from species to species, the general characterization is common for all mammals. Auditory stimuli are mechanically transmitted through the external auditory canal, tympanic membrane, and ossicular chain to the inner ear.

Many studies have proposed various drugs and agents as potential therapeutic interventions in ototoxicity, ARHL (Age Related Hearing Loss) and noise damage. These broadly fall into two categories: those aimed at inhibiting specific parts of the cell death pathways, and those aimed at interrupting the signaling mechanism leading to apoptosis and necrosis. Several strategies of intervention are based on the reduction of ROS (Reactive Oxygen Species) by using anti-oxidant drugs such as N-acetylcysteine (NAC), already used in some clinical trials.

In the last years many studies concerning hair cell regeneration were conducted. Many of them have shown that supporting cells of the mammalian organ of Corti can be induced to division after specific gene manipulation. Recent evidence suggests that the postnatal mammalian inner ear contains progenitor cells, which are able to proliferate *in vitro* or form otospheres and differentiate into multiple phenotypes.

Rehabilitative measures traditionally used are based on technical solutions, primarily using hearing aids to amplify and filter incoming sounds signals, while in cases of more severe impairment an option is offered by the cochlear prosthesis, which directly stimulates the auditory neurons. Altering expression of specific genes responsible for the differentiation of hair cells (Mizutari K et al., Neuron 2013; 77(1):58-69) or stem cell therapy (Okano T et al., Trends Amplif. 2012;16(1):4-18) is likely the solution for hearing function restoration. Methods or strategies for hair cell protection able to delay the degeneration process are also actively pursued. However an effective clinical treatment has not yet been found.

The NGF family of neurotrophins is a class of emerging molecules required for the inner ear innervation in mammals (Ernfors P et al., Neuron 1995;14:1153-64); their potential therapeutic application in humans is actively under evaluation. This family includes the nerve growth factor (NGF), the brain-derived neurotrophic factor (BDNF) and the neurotrophins 3 and 4 (NT3, NT4). These four molecules share a similar sequence and structure, as they all descend from a common ancestral gene, and all four are active in directing neurons growth and differentiation during development and beyond (Huang EJ et al., Annu Rev Neurosci 2001;24:677-736). Each neurotrophin binds to a specific subtype of Trk receptor with high affinity, and some low affinity cross binding to other Trk receptors and to p75 receptor also occurs.

The brain-derived neurotrophic factor (BDNF) acts on certain neurons of the central nervous system and the peripheral nervous system, helping to support the survival of existing neurons, and encourage the growth and differentiation of new neurons and synapses.

Neurotrophin 3 is a protein growth factor which has activity on certain neurons of the peripheral and central nervous system; it helps to support the survival and differentiation of existing neurons, and encourages the growth and differentiation of new neurons and synapses.

Neurotrophin 4 is a neurotrophic factor that signals predominantly through the TrkB receptor tyrosine kinase.

NGF is a polypeptide composed of three subunits, α, β and γ. The β-subunit itself is a homodimer of peptides composed by 118 amino acids and is responsible for the full biological activity of NGF. Its structure is well preserved among different species, with 90% homology between murine and human NGF. NGF regulates growth and survival of nerve cells, profoundly affecting the development of both young and adult nervous system.

In adults, NGF was shown to be effective in pain/hyperalgesia, as well as in several eye diseases, such as neurotrophic keratitis, optic nerve transection, ocular hypertension and retinal detachment. Said data have been reported mainly for NGF extracted from murine salivary glands, even these preparations are heterogeneous mixtures of different dimers.

Recently, human NGF was synthesized using genetic engineering techniques (Iwane et al., Biochem. Biophys. Res. Commun., 171: 116, 1990; EP099188B1, WO2013/092776), with the advantage to obtain a more homogeneous form of protein to be administered to patients. Various animal models were used to stimulate and investigate the morphological and physiologic effects of the insults to the inner ear. When administered to deafened cochlea in animal models, neurotrophins, showed to be able to dramatically improve spiral ganglion neuron survival and to stimulate peripheral auditory fiber regrowth.

Furthermore, patients affected by blast-induced hearing loss and treated with NGF by intramuscular injection showed a hearing improved when early treated (S.Q. Zhai, N. YU. Eur. Rev. Med. Pharm. Sci., 2015;19:3146-51). A therapeutic effect was observed following NGF-point injections in the treatment of nervous deafness and tinnitus (Shanxi et al., J. Trad Chinese Med 2009;29:39-42).

Although the parenteral administration of NGF, in particular by intramuscular and intravascular route, is needed when a prolonged NGF action is wanted, said route of administration does not solve the main issue to reach high doses of NGF in the inner ear, in particular to achieve a therapeutic and efficacious intralabyrinth concentration. Furthermore, several known strategies for NGF delivery to the inner ear are known, that include systemic and intravenous administration, but these routes are not selective, and therapeutic concentrations are difficult to be achieved inside the inner ear.

There is therefore the need of a more specific and localized treatment able to reach the inner ear with a concentration of drug sufficiently high to guarantee the therapeutic efficacy of NGF.

### DEFINITIONS

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The term "pharmaceutically acceptable excipient" herein refers to a substance devoid of any pharmacological effect of its own and which does not produce adverse reactions when administered to a mammal, preferably a human. Pharmaceutically acceptable excipients are well known in the art and are disclosed, for instance in the Handbook of Pharmaceutical Excipients, sixth edition 2009, herein incorporated by reference.

The term "simultaneous, separate or sequential administration" herein refers to administration of the first and second compound at the same time or in such a manner that the two compound act in the patient's body at the same time or administration of one compound after the other compound in such a manner to provide a therapeutic effect.

The terms "approximately" and "about" herein refers to the range of the experimental error, which may occur in a measurement.

The terms "comprising", "having", "including" and "containing" are to be construed open-ended terms (i.e. meaning "including, but not limited to") and are to be considered as providing support also for terms as "consist essentially of", "consisting essentially of", "consist of" or "consisting of".

The terms "consist essentially of", "consisting essentially of" are to be construed as semi-closed terms, meaning that no other ingredients which materially affects the basic and novel characteristics of the invention are included (optional excipients may thus included).

The terms "consists of", "consisting of" are to be construed as closed terms.

The term "intratympanic administration" means a surgical procedure in which a small amount of a drug is injected directly into the middle ear through the tympanic membrane. It is usually performed at the postero-inferior quadrant of the tympanic membrane which is at the level of the round window, "the gate to the inner ear", because it hosts a membrane permeable to some drugs.

### DESCRIPTION OF THE FIGURES

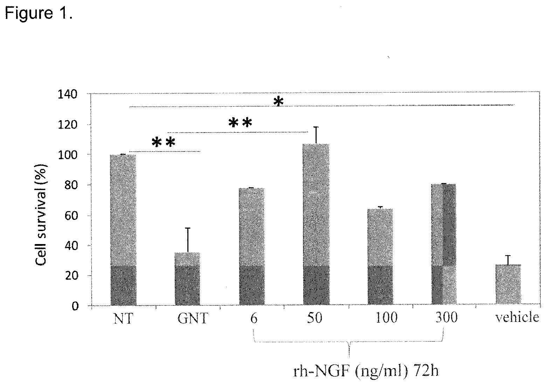

Figure 1. Cell survival after rhNGF treatment of damaged cochlea.

### DESCRIPTION OF THE INVENTION

It was surprisingly found that the local neurotrophin delivery approach including the intratympanic administration, that limits systemic exposure, is particularly promising in the treatment of hearing loss, because it allows the neurotrophin to reach adequate therapeutic levels into the inner ear, particularly in the intra labyrinthique region, faster and at higher concentrations.

This route of administration provides for better results than other common routes, such as intravenous or oral administration. The obtained results are imputable to the neurotrophin activity in restoring hair cells viability after gentamicin treatment, and to the transtympanic administration leading to a good distribution of neurotrophin into the cochlea.

In particular, the experimental data reported in the present invention shows that the transtympanic administration of rhNGF is able to regenerate the inner ear tissue, particularly the inner ear epithelial hair cells, and therefore represents an improvement over the existing treatments and an alternative to corticosteroid treatment both by oral and parenteral administration.

Therefore, a first embodiment of the present invention relates to the use of a neurotrophin in the treatment of hearing disorders, characterized in that said neurotrophin is administered by intratympanic route.

In a preferred embodiment of the present invention said neurotrophin is selected from NGF, brain-derived neurotrophic factor (BDNF), neurotrophin 3 and neurotrophin 4, more preferably NGF.

Preferably, said hearing disorders are selected from sudden deafness, blast-induced hearing loss, ototoxicity, ARHL (age related hearing loss) and noise damage.

In a preferred embodiment, said neurotrophin is administered by means of a composition further comprising at least one pharmaceutically acceptable excipient. Preferably, the administered neurotrophin is NGF, brain-derived neurotrophic factor (BDNF), neurotrophin 3 and neurotrophin 4, more preferably NGF.

For the purpose of the present invention pharmaceutically acceptable excipient suitable for intratympanic administration are selected from hyaluronic acid, polyvinyl alcohol (PVA), glycerol, poly-lactic-co-glycolic acid (PLGA) and PEG400, preferably hyaluronic acid, polyvinyl alcohol or glycerol.

In a preferred embodiment of the present invention, said composition is a solution, a suspension or a gel.

Preferably, when the composition is a gel, said gel is selected from a thermosensitive gel or an adhesive sol-gel transition hydrogels. More preferably said gels include Pluronic F127 and Hyaloronic acid (HA).

In a further embodiment the composition of the present invention can be administered simultaneously, separately or sequentially in combination with at least one further active principle, also following different route of administration for each active principle.

In a preferred embodiment said at least one further active principle is a steroid, selected from dexametasone, methylprednisolone and prednisolone.

Preferably, said further active principle can be administered orally, intramuscularly or topically.

In a further embodiment, the composition of the present invention contains from 1 µg to 1.2 mg of neurotrophin, preferably from 10 µg to 200 µg of neurotrophin, more preferably from 2 µg to 20 µg of neurotrophin.

Preferably said neurotrophin is NGF, brain-derived neurotrophic factor (BDNF), neurotrophin 3 and neurotrophin 4, more preferably NGF.

According to a preferred embodiment, the composition of the present invention contains from 1 µg 1.2 mg of NGF, preferably from 10 µg to 200 µg of NGF, more preferably from 2 µg to 20 µg of NGF.

When the composition of the present invention is in form of a suspension the concentration of neurotrophin is from 1 µg/ml to 1.2 mg/ml, preferably from 10 µg/ml to 200 µg/ml, more preferably from 2 µg/ml to 20 µg/ml of neurotrophin.

Preferably said neurotrophin is NGF, brain-derived neurotrophic factor (BDNF), neurotrophin 3 and neurotrophin 4, more preferably NGF.

In a more preferred embodiment in said suspension the concentration of NGF is from 1 µg/ml to 1.2 mg/ml, preferably from 10 µg/ml to 200 µg/ml, more preferably from 2 µg/ml to 20 µg/ml of NGF.

When the composition of the present invention is in form of a solution the concentration of neurotrophin is from 1 µg/ml to 1.2 mg/ml, preferably from 10 µg/ml to 200 µg/ml, more preferably from 2 µg/ml to 20 µg/ml of neurotrophin.

Preferably said neurotrophin is NGF, brain-derived neurotrophic factor (BDNF), neurotrophin 3 and neurotrophin 4, more preferably NGF.

When the composition of the present invention is in form of a solution the concentration of NGF is from 1 µg/ml to 1.2 mg/ml, preferably from 10 µg/ml to 200 µg/ml, more preferably from 2 µg/ml to 20 µg/ml of NGF.

In a further embodiment the neurotrophin of the present invention is of murine or human origin or it is a human recombinant neurotrophin.

Preferably, said neurotrophin is a NGF of murine or human origin or is a human recombinant NGF.

According to a preferred embodiment, the composition for use of the present invention is administered daily, preferably one or more time a day.

Preferably, said composition for use is administered from one to four doses a day, for at least 4 weeks.

According to the invention, the composition of the present invention may be administered to humans, intended to comprise both adults and the "pediatric population" (where the term "pediatric population" is understood as the part of the population ranging from birth to eighteen years of age).

The following examples are included to increase the understanding of the invention, without having any limiting effect of the invention.

### EXPERIMENTAL DATA

The following mixtures according to the present invention were prepared.

### Formulation 1

### Microparticles slow release

The PLGA/NGF microparticles have been prepared as follow. The yield is of 74.31 % with a theoretical amount of 0.0088% (mg NGF / 100mg mp). A 100 µl aqueous solution containing NGF (50 µl), HSA (1.99 mg) and PEG400 (50 µl) (1: 40: 1 ratio, vol./weight/vol.) was emulsified for 1 minute in 2.5 ml of 20% solution of PLGA 752H in Ethyl Acetate containing 0.5 g of polymer by means of an Ultraturrax homogenizer T25 Basic (IKA), with 8G probe, set at 17500 rpm. The obtained emulsion (A1/O) was rapidly added to 10 ml of an aqueous solution PVA 1% p/v (Mowiol® 40-88) mixed with 300 µl of ethyl acetate and homogenized at 12600 rpm for 30 minutes. The resulting double emulsion (A1/O/A2) was left under electromagnetic stirring for 3 hours at room temperature using an RW20D (IKA) rod stirrer, set at 500 rpm, to facilitate evaporation of the organic solvent and precipitation of PLGA particles. The suspension was collected in a 50 ml tube and centrifuged at 7000 rpm for 15 min at 4°C.

The isolated microparticles were washed 4 times with 50 ml of MilliQ water, centrifuged (7000 rpm, 4°C, 15 minute), and then resuspended in 3 ml of 2% w/v aqueous trealose solution. The obtained suspension was transferred into 2 double silicone glass vials, frozen at -80°C, then subjected to a 24 hour lyophilization cycle.

**Table 1. Microparticles components**

| **Components** | **Quantity** |
|---|---|
| **rhNGF** | **0.0495 mg** |
| **PLGA 752H** | **2.5 ml** |
| **PEG 400** | **0.05 ml** |
| **HSA** | **1.99 mg** |
| **Ethyl acetate** | **2.8 ml** |
| **Polyvinyl alcohol 40-88** | **10 ml** |
| **Trealose** | **3 ml** |

### 1. In vitro experiment

Cochlear culture represents the standard model to test ability of compound possibly effective in reducing or preventing hearing loss. In the experiment here described, cochlea were extracted from newborn mice (from day 5 to day 15) and damaged with gentamicin treatment (50µg/ml for 14 hours), Then rhNGF was used at different concentration (6-50-100-300 ng/ml) for 72h. Cell survival was evaluated by Hoechst staining.

Non treated cells (NT), damaged and not treated cells (GNT) and vehicle treated cells were used as controls.

### Results

As reported in Figure 1, the rhNGF treatment of damaged cochlea led to a statistically significant increase in cell survival.

In particular, the data obtained in the experiment demonstrated the rhNGF ability in restoring hair cell viability following damage due to gentamicin exposure in organotypic cultures of the cochlea of mice.

### 2. Pharmacokinetic of rhNGF after Transtympanic administration in guinea pig

The trans-tympanic injection was carried out on anesthetized animals using a specific endoscope to limit the damage of the tympanic membrane. The drug was administered in the left middle ear of the Guinea Pig, laying on its right side on a heating pad. Perilymph was collected after 0.5, 2, 4, 6 and 24 hours post injection Pharmacokinetic profile of rhNGF after single intravenous (iv) (1.2 mg/kg) and transtympanic (TT) (30 µg/mL) administration was studied in female Albino Hartley guinea pigs.

rhNGF was solubilized in saline buffer and diluted in artificial perilymph to obtain a final concentration.

The trans-tympanic injection was carried out on anesthetized animals using a specific endoscope to limit the damage of the tympanic membrane. The drug was administered in the left middle ear of the Guinea Pig, laying on its right side on a heating pad.

5µL of cochlear fluid from 5 guinea pigs/time were collected at 0.5, 2, 4, 6 and 24h after administration.

rhNGF was detected in perilymph (ELISA).

In Table 2 the main pharmacokinetic parameters of rhNGF obtained after single i.v. and TT administration are shown.

**Table 2. Pharmacokinetic parameters of rhNGF after single i.v. and TT administration**

| rhNGF | TT | IV |
|---|---|---|
| DOSE | 30µg/mL | 1,2mg/kg |
| Cmax (ng/mL) | 711,927 | 17,3877 |
| Tmax (h) | 4 | 2 |
| AUClast (µg/mL*min) | 305,595 | 11,4342 |
| T_{1/2}(h) | 17,4846 | 10,1469 |

These data showed that transtympanic injection (TT) is better than i.v. since it results in a Cmax 40-fold and an AUCₜₒₜ (the peak concentration of a drug after administration in perilymph)(Area Under the Curve represents the total drug exposure over time given by summa of AUC last and extra, respectively the AUC of the last dosed time and the AUC of all dosed times after 24 hours from drug exposure) (544,799µg/mL*min) 38-fold higher with a t_{1/2} roughly 2-fold longer, despite rhNGF dose 40-fold lower in TT than in i.v. administration.

## Claims

1. Neurotrophin for use in the treatment of hearing disorders, **characterized in that** it is administered by intratympanic route.

2. Neurotrophin for use according to claim 1, **characterized in that** said neurotrophin is selected from NGF, brain-derived neurotrophic factor (BDNF), neurotrophin 3 and neurotrophin 4, preferably NGF.

3. Neurotrophin for use according to claims 1 or 2, **characterized in that** said disorders are selected from sudden deafness, blast-induced hearing loss, ototoxicity, ARHL (age related hearing loss) and noise damage.

4. Neurotrophin for use according to any of the previous claims, **characterized in that** it is administered by means of a composition further comprising at least a pharmaceutically acceptable excipient.

5. Neurotrophin for use according to claim 4, **characterized in that** said pharmaceutically acceptable excipient is selected from hyaluronic acid, polyvinyl alcohol, glycerol, poly-lactic-co-glycolic acid and PEG400, preferably hyaluronic acid, polyvinyl alcohol or glycerol.

6. Neurotrophin for use according to any of the previous claims, **characterized in that** said composition is a solution, a suspension or a gel.

7. Neurotrophin for use according to any of the previous claims, **characterized in that** said composition can be administered simultaneously, separately or sequentially in combination with a further active principle.

8. Neurotrophin for use according to claim 7, **characterized in that** said further active principle is a steroid, selected from dexamethasone, methylprednisolone and prednisolone.

9. Neurotrophin for use according to any of the previous claims, **characterized in that** said composition contains from 1 µg to 1.2 mg of NGF, preferably from 10 µg to 200 µg of NGF, more preferably from 2 µg to 20 µg of NGF.

10. Neurotrophin for use according to claim 6, **characterized in that** said solution contains a concentration of NGF ranging from 1 µg/ml to 1.2 mg/ml, preferably from 10 µg/ml to 200 µg/ml, more preferably from 2 µg/ml to 20 µg/ml.

11. Neurotrophin for use according to claim 6, **characterized in that** said suspension contains a concentration of NGF ranging from 1 µg/ml to 1.2 mg/ml, preferably from 10 µg/ml to 200 µg/ml, more preferably from 2 µg/ml to 20 µg/ml.

12. Neurotrophin for use according to any of the previous claims, **characterized in that** it is of murine or human origin or it is a human recombinant neurotrophin.

13. Neurotrophin according to claim 12, **characterized in that** said neurotrophin is a NGF of murine or human origin or it is a human recombinant NGF.

14. Neurotrophin for use according to any of the previous claims, **characterized in that** said composition is administered daily, preferably one or more time a day.
